# EUROPEAN PATENT SPECIFICATION

(11) **EP 3 110 407 B1**
(45) Date of publication and mention of the grant of the patent: **01.12.2021**
(21) Application number: 15755017.9
(22) Date of filing: 25.02.2015
(51) Int. Cl.: B01J 31/06, B01J 35/00, B01J 23/42, B01J 23/50, B01J 23/52, B01J 23/75, B01J 37/03, B01J 37/16, C07C 209/36, A61K 9/107, A61K 47/32

(54) **METAL-POLYMER COMPOSITE NANOPARTICLES**
METALL-POLYMER-KOMPOSITNANOPARTIKEL
NANOPARTICULES COMPOSÉS METAL-POLYMÈRE

(30) Priority: 26.02.2014 US 201461944784 P; 27.08.2014 US 201462042515 P
(43) Date of publication of application: 04.01.2017
(73) Proprietor: The Trustees of Princeton University, Princeton, NJ 08544 (US)
(72) Inventor: PRUD'HOMME, Robert K., Lawrenceville, New Jersey 08648 (US); PRIESTLEY, Rodney D., Princeton, New Jersey 08540-3904 (US); LIU, Rui, Princeton, New Jersey 08540 (US); SOSA, Chris, Dover, New Jersey 07801 (US)
(74) Representative: dompatent von Kreisler Selting Werner - Partnerschaft von Patent- und Rechtsanwälten mbB
(86) International application number: PCT/US2015/017590
(87) International publication number: WO 2015/130835

(56) References cited:
- WO-A1-2013/053598
- US-A1- 2003 013 799
- US-A1- 2008 260 851
- US-A1- 2010 330 368
- US-A1- 2010 330 368
- US-A1- 2011 213 066
- US-A1- 2012 041 150
- US-A1- 2012 052 097
- US-A1- 2013 171 208
- US-B1- 8 288 001
- US-B2- 7 112 369
- US-B2- 7 348 365
- US-B2- 8 137 699
- US-B2- 8 137 699
- MAYER A B R ET AL: "Transition metal nanoparticles protected by amphiphilic block copolymers as tailored catalyst systems", COLLOID AND POLYMER SCIENCE ; KOLLOID-ZEITSCHRIFT UND ZEITSCHRIFT FÜR POLYMERE, SPRINGER, BERLIN, DE, vol. 275, no. 4, 1 April 1997 (1997-04-01) , pages 333-340, XP019712855, ISSN: 1435-1536
- QINGJIE LUO ET AL: "Controlling the Location of Nanoparticles in Colloidal Assemblies of Amphiphilic Polymers by Tuning Nanoparticle Surface Chemistry", ACS MACRO LETTERS, vol. 2, no. 2, 11 January 2013 (2013-01-11), pages 107-111, XP055416332, ISSN: 2161-1653, DOI: 10.1021/mz3006044
- Oskar Nuyken ET AL: "Polymers for Micellar Catalysis" In: "Polymeric materials in organic synthesis and catalysis", 1 January 2003 (2003-01-01), WILEY-VCH, WEINHEIM (DE), XP055415485, ISBN: 978-3-527-30630-5 pages 277-304, * pages 283-286 *
- Arne Thomas: "Micelle Formation - Lecture Colloidal Phenomena", Journal of Polymer Science Part A: Polymer Chemistry, 11 July 2013 (2013-07-11), pages 1-50, XP055415590, Retrieved from the Internet: URL:https://www.mpikg.mpg.de/886719/Micell eFormation.pdf [retrieved on 2017-10-13] -& Anon: "Firefox Page Info: https://www.mpikg.mpg.de/886719/MicelleFor mation.pdf", , 11 July 2013 (2013-07-11), XP055415585, Retrieved from the Internet: URL:https://www.mpikg.mpg.de/886719/Micell eFormation.pdf [retrieved on 2017-10-13]
- AIZAWA M ET AL: "Block Copolymer-Templated Chemistry on Si, Ge, InP, and GaAs Surfaces", JOURNAL OF THE AMERICAN CHEMICAL SOCIETY, AMERICAN CHEMICAL SOCIETY, US, vol. 127, no. 25, 29 June 2005 (2005-06-29), pages 8932-8933, XP002610248, ISSN: 0002-7863, DOI: 10.1021/JA052281M [retrieved on 2005-06-01]
- RUI LIU ET AL: "A one-step and scalable production route to metal nanocatalyst supported polymer nanospheres via flash nanoprecipitation", JOURNAL OF MATERIALS CHEMISTRY A: MATERIALS FOR ENERGY AND SUSTAINABILITY, vol. 2, no. 41, 22 September 2014 (2014-09-22), pages 17286-17290, XP055416197, GB ISSN: 2050-7488, DOI: 10.1039/C4TA04036H
- DICKINSON, E ET AL.: 'Food Emulsions and Foams: Stabilization by Particles.' CURRENT OPINION IN COLLOID & INTERFACE SCIENCE vol. 15, no. 1, 2010, XP026896477
- NIEMINEN, JJ ET AL.: 'Hydrogen evolution catalyzed by electrodeposited nanoparticles at the liquid/liquid interface.' CHEMICAL COMMUNICATIONS vol. 47, no. 19, 2011, XP055358016
- ERHARDT, R ET AL.: 'Amphiphilic Janus Micelles with Polystyrene and Poly(methacrylic acid) Hemispheres.' JOURNAL OF THE AMERICAN CHEMICAL SOCIETY vol. 125, no. 11, 2003, XP055358020
- KIYONO, Y ET AL.: 'Preparation and Structural Investigation of PMMA-Polystyrene 'Janus Beads' by Rapid Evaporation of an Ethyl Acetate Aqueous Emulsion.' E-JOUMAL OF SURFACE SCIENCE AND NANOTECHNOLOGY. vol. 10, 2012, pages 360 - 366, XP055222187
- JANG, SG ET AL.: 'Bicontinuous block copolymer morphologies produced by interfacially active, thermally stable nanoparticles.' MACROMOLECULES vol. 44, no. 23, 2011, pages 9366 - 9373, XP055222191
- None

## Description

This invention was made with government support under Grant No. DMR-0819860 awarded by the National Science Foundation. The government has certain rights in the invention.

### FIELD OF THE INVENTION

The present invention relates to metal- polymer composite nanoparticles and processes of making them.

### BACKGROUND OF THE INVENTION

In Pierre Gilles de Gennes's 1991 Nobel Laureate speech titled "Soft Matter" he introduced the concept of Janus particles, which are anisotropically structured particles containing two distinct regions of material or functionality. Their development can be considered in the context of the scientific and technological development of other chemically anisotopically structured materials, such as surfactants and block copolymers. The ability to synthesize surfactants at scale and in cost effective ways has led to the current surfactant market. The ability to synthesize block copolymers at scale and in cost effective ways has led to the current market for thermoplastic elastomers based on block copolymers.

Janus colloids can be assembled from a broad variety of building blocks ranging from metals to polymers (Yoon, J. et al., Amphiphilic colloidal surfactants based on electrohydrodynamic co-jetting, ACS Appl. Mater. Interfaces, 2013, 5, 11281-7; Glaser, N. et al., Janus particles at liquid-liquid interfaces, Langmuir 2006, 22, 5227-9). The breadth of material properties exhibited by polymers as well as their ability to phase separate can be useful for the generation of Janus colloids.

Colloids possessing patterned or structured surface domains of differing chemical composition can serve as nanoscale building blocks for the design of materials with molecular scale features (Walther, A. et al., Janus particles. Soft Matter 2008, 4, 663―668; Walther, A. et al. Janus Particles: Synthesis, Self-Assembly, Physical Properties, and Applications, Chem. Rev. 2013, 113, 5194-5261; Samuel, A.Z. et al., Self-Adapting Amphiphilic Hyperbranched Polymers, Macromolecules 2012, 45, 2348-2358). The functionality of such particles can depend on the spatial topology and molecular properties of surface domains (Walther, A. et al., Janus particles, Soft Matter 2008, 4, 663-668; Walther, A. et al., Janus Particles: Synthesis, Self-Assembly, Physical Properties, and Applications, Chem. Rev. 2013, 113, 5194―5261).

Janus nanocolloids can assemble into higher-order superstructures when induced to by various environmental stimuli (Walther, A. et al., Janus particles, Soft Matter 2008, 4, 663-668; Chen, Q. et al., Directed self-assembly of a colloidal kagome lattice, Nature 2011, 469, 381-384). They can, for example, organize under magnetic (Smoukov, S.K. et al., Reconfigurable responsive structures assembled from magnetic Janus particles, Soft Matter 2009, 5, 1285-1292) or electric fields (Gangwal, S. et al., Dielectrophoretic Assembly of Metallodielectric Janus Particles in AC Electric Fields, Langmuir 2008, 24, 13312-13320) to form patterned chains on solid substrates, undergo complex translational (Gangwal, S. et al., Induced-Charge Electrophoresis of Metallodielectric Particles, Phys. Rev. Lett. 2008, 100, 058302) and rotational (Gangwal, S. et al. Induced-Charge Electrophoresis of Metallodielectric Particles, Phys. Rev. Lett. 2008, 100, 058302) motion in alternating fields (Squires, T.M. et al., Breaking symmetries in induced-charge electro-osmosis and electrophoresis, J. Fluid Mech. 2006, 560, 65-101), migrate to the interface between two immiscible fluids in order to decrease the surface tension of macroscopic emulsions (Yoon, J. et al., Amphiphilic colloidal surfactants based on electrohydrodynamic co-jetting, ACS Appl. Mater. Interfaces 2013, 5, 11281-7), and uniquely interact with cellular interfaces in order to facilitate the absorption of imaging or therapeutic agents (Gao, Y. et al., How half-coated janus particles enter cells, J. Am. Chem. Soc. 2013, 135, 19091-4).

The interest in multi-faced nanocolloid applications has outstripped the ability to produce commercial-scale materials, hindering the development of new technologies (Samuel, A.Z. et al., Self-Adapting Amphiphilic Hyperbranched Polymers, Macromolecules 2012, 45, 2348-2358; Jang, S.G. et al., Striped, ellipsoidal particles by controlled assembly of diblock copolymers, J. Am. Chem. Soc. 2013, 135, 6649―57; Erhardt, R. et al., Amphiphilic Janus micelles with polystyrene and poly(methacrylic acid) hemispheres, J. Am. Chem. Soc. 2003, 125, 3260-3267; Roh, K. et al., Biphasic Janus particles with nanoscale anisotropy, Nat. Mater. 2005, 4, 759-763; Yamashita, N. et al., Preparation of hemispherical particles by cleavage of micrometer-sized, spherical poly(methyl methacrylate)/polystyrene composite particle with Janus structure: effect of molecular weight, Colloid Polym. Sci. 2013, 292, 733―738).

The scalability of processes for forming and comprehensive control over particle morphology of Janus particles is a challenge (Chang, E.P. et al., Membrane Emulsification and Solvent Pervaporation Processes for the Continuous Synthesis of Functional Magnetic and Janus Nanobeads, Langmuir 2012, 28, 9748-9758; Wang, Y. et al., Colloids with valence and specific directional bonding, Nature 2012, 491, 51-5; Walther, A. et al., Janus discs, J. Am. Chem. Soc., 2007, 129, 6187-98).

Metal nanoparticles (Burda, C. et al., Chem. Rev. 2005, 105, 1025) are typically unstable and tend to sinter into larger species. A suitable carrier is needed to prevent the aggregation of metal nanoparticles (Astruc, D. et al., Angew. Chem. Int. Ed. 2005, 44, 7852.; Na, H.B. et al., Adv. Mater. 2009, 21, 2133). Polymeric matrices (Scott, R.W. et al., J. Am. Chem. Soc. 2004, 126, 15583; Anderson, R.M. et al., ACS Nano 2013, 7, 9345.; Peng, X. et al., Chem. Soc. Rev. 2008, 37, 1619), latex particles (Sun, Q. et al., Langmuir 2005, 21, 5812; Mohammed, H.S. et al., Macromol. Rapid Commun. 2006, 27, 1774; Wong, J.E. et al., J. Colloid Interface Sci. 2008, 324, 47; Ganesan, V. et al. Soft Matter 2010, 6, 4010; Liu, R. et al., ACS Appl. Mater. Interfaces 2013, 5, 9167) have been studied for the immobilization of metal nanoparticles. (Mei, Y. et al, Chem. Mater. 2007, 19, 1062; Schrinner, M. et al., Adv. Mater. 2008, 20, 1928; Lu, Y. et al., Macromol Rapid Comm. 2009, 30, 8o6.; Wunder, S. et al., J. Phys. Chem. C 2010, 114, 8814; Shenhar, R. et al., Adv. Mater. 2005, 17, 657; Grzelczak, M. et al., ACS Nano 2010, 4, 3591). Nanoparticles, such as magnetic nanoparticles (Krack, M. et al., J. Am. Chem. Soc. 2008, 130, 7315) and quantum dots (Diaz, A. at al., Am. Chem. Soc. 2013, 135, 3208) can be encapsulated in various polymer assemblies to form multifunctional materials (Mai, Y. et al., J. Am. Chem. Soc. 2010, 132, 10078.; Jang, S.G. et al., J. Am. Chem. Soc. 135, 6649; Bae, J. et al., Adv. Mater., 2012, 24, 2735; Chen, H. et al., Chem. Phys. Chem. 2008, 9, 388; Li, W.K. et al., Angew. Chem., Int. Ed. 2011, 50, 5865; Kang, Y. et al., Angew. Chem., Int. Ed. 2005, 44, 409; Kim, B.S. et al., Nano Lett. 2005, 5, 1987; Hickey, R.J. et al., J. Am. Chem. Soc. 2011, 133, 1517; Luo, Q. et al., ACS Macro Lett. 2013, 2, 107).

Oskar Nuyken et al.: "Polymers for Micellar Catalysis" In: "Polymeric materials in organic synthesis and catalysis", WILEY-VCH, WEINHEIM (DE), 2003, pages 278-286 discloses amphiphilic block copolymer micelles for the general purpose of micellar catalysis, especially for hydrophobic substrates in in aqueous media, using immobilized metal complexes or hydrophobic complexes confined in the hydrophobic core. Likewise disclosed are micelles comprising amphiphilic block copolymer encapsulating within the core region metal nanoparticles for the same catalysis purpose, including in aqueous media. Nuyken mentions the block copolymer PS-b-PVP, Pd, Au, Pd/Au for catalytic uses, metal nanoparticles being in a core region, and performing reactions in non-aqueous, optionally polar environments (toluene, THF, cyclohexane).

### SUMMARY

Methods according to the invention use Flash NanoPrecipitation (FNP) to produce metal-polymer composite Janus particles as defined in claim 1, using processes that confine the volume for phase separation. In comparison to existing processes, FNP is a single-step, low energy, continuous, and rapid process that can be used to create polymer:polymer and polymer:inorganic nanoparticles.

Thus polystyrene-block-poly(vinylpyridine) (PS-b-PVP) in for example tetrahydrofuran (THF), aqueous metal ion salts, and reducing agent solutions (for example, of NaBH₄) can be employed as polymer stream, non-solvent stream, and collection solution, respectively to obtain uniform metal nanoparticles grown on polymer nanospheres through a one-step FNP process. The particle size and metal nanoparticle loading amount can be tuned by changing the preparation parameters (e.g., feeding amount, feeding speed, concentration of polymer in the feed, mixing rate).

Flash Nano Precipitation (FNP) achieves rapid solvent displacement by means of high intensity mixing geometries. Amphiphilic block copolymer chains dissolved in a solvent are mixed with a non-solvent, e.g., typically water, to precipitate and assemble as nanoparticles. For example, metal (e.g. gold (Au) or platinum (Pt)) nanoparticles - nanosphere polymer composites can be generated through FNP. Thus, uniform metal-nanosphere polymer composites with 2-3 nm metal colloids (nanocrystals) decorating the PVP corona on the nanosphere (i.e., PVP polymer constituents on the surface of the nanosphere and PVP polymers and portions of polymers radiating out from surface into the solution) can be obtained with overall particle size and metal nanoparticle arrangement tunable by varying the process parameters. In an exemplary use, the obtained composites show high catalytic ability and stability in the reduction of 4-nitrophenol.

A method according to the invention as defined in claim 1 is forming a metal-polymer composite nanoparticle by dissolving a polymer in a first solvent at a first concentration to form a polymer solution, dissolving a metal salt in a second solvent at a second concentration to form a metal salt solution, and mixing the polymer solution with the metal salt solution to form the metal-polymer composite nanoparticle with a surface, by means of flash nanoprecipitation (FNP). The metal is concentrated at the surface. The second solvent is a nonsolvent for the polymer. The polymer is an amphiphilic block copolymer, being polystyrene-block-poly(vinylpyridine) (PS-b-PVP). The metal can be gold (Au), platinum (Pt), silver (Ag), palladium (Pd), copper (Cu), cobalt (Co), and/or iron (Fe). The mixing of the polymer solution with the metal salt solution includes mixing with a collection solution. The collection solution includes a reducing agent. For example, the reducing agent can be selected from lithium aluminum hydride (LiAlH₄), compounds containing the Sn²⁺ ion, such as tin(II)chloride (SnCl₂), compounds containing the Fe²⁺ ion, such as iron (II) sulfate (FeSO₄), oxalic acid, formic acid, ascorbic acid, sulfite compounds, phosphites, hydrophosphites, phosphorous acid, dithiothreitol (DTT), tris(2-carboxyethyl)phosphine HCl (TCEP), and/or carbon. For example, the reducing agent can be sodium borohydride (NaBH₄). The collection solution includes a stabilizer, for example, sodium dodecyl sulfate (SDS).

In an embodiment according to the invention, a metal-polymer composite nanoparticle includes a core and a shell that surrounds the core. The core includes the defined polymer, and the shell includes the defined polymer and one or more of the defined metals. In a method according to the invention, the metal-polymer composite nanoparticle is used to catalyze a chemical reaction. For example, the chemical reaction catalyzed can be between two immiscible phase liquids.

### BRIEF DESCRIPTION OF THE FIGURES

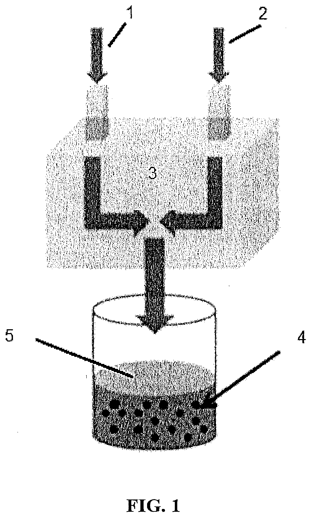

Figure 1 shows a schematic of the Flash NanoPrecipitation (FNP) method.

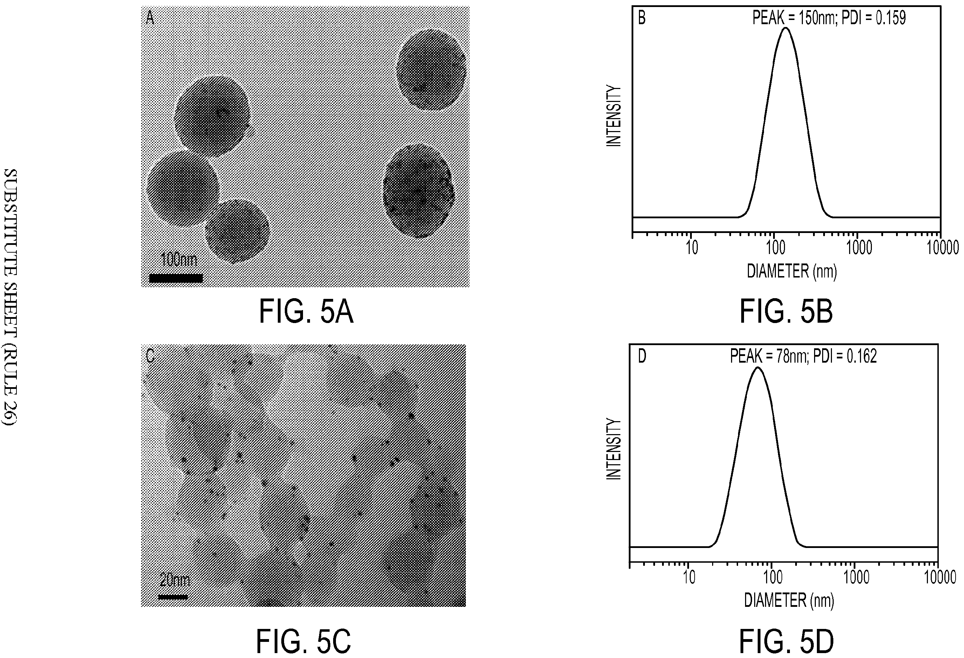

Figures 5A and 5B show a tunneling electron microscopy (TEM) image and dynamic light scattering (DLS) data, respectively, for metal-polymer hybrid nanoparticles prepared with a solvent stream of 6 mg/mL polystyrene-block-poly(vinylpyridine) (PS-b-PVP) in THF and a non-solvent stream of 0.45 mg/mL chloroauric acid (HAuCl₄) in H₂O. Figures 5C and 5D show a tunneling electron microscopy (TEM) image and dynamic light scattering (DLS) data, respectively, for metal-polymer hybrid nanoparticles prepared with a solvent stream of 1 mg/mL polystyrene-block-poly(vinylpyridine) (PS-b-PVP) in THF and a non-solvent stream of 0.15 mg/mL chloroauric acid (HAuCl₄) in H₂O.

### DETAILED DESCRIPTION

The terms "particle", "nanoparticle", "colloid", and "nanocolloid" are used interchangeably herein, unless another meaning is indicated by the context. The term "Janus" refers to a particle having two distinct surfaces, for example, having two surfaces of different polymers. The term "Janus" can also refer to a characteristic of such a particle or group of particles, such as "Janus morphology" or "Janus phase".

### Flash NanoPrecipitation

Flash NanoPrecipitation (FNP) can be used for the production of organic and organic/inorganic nanoparticles. The mean particle diameter of these nanoparticles can be in the range of from 30 to 2000 nm, for example, from about 50 to 800 nm. For example, the mean particle diameter of these nanoparticles can be from about 10, 20, 30, 50, 60, 100, 200, 300, 500, 800, 1000, 1200, 1500, 2000, 4000, 5000, 6000, or 10,000 nm to about 20, 30, 50, 60, 100, 200, 300, 500, 800, 1000, 1200, 1500, 2000, 4000, 5000, 6000, 10,000, or 20,000 nm. FNP can form particles of narrow size distribution. For example, of the nanoparticles formed, at least 90% can have a diameter less than 800 nm, and at most 10% can have a diameter less than 50 nm. For example, of the nanoparticles formed, at least 90% can have a diameter less than 50,000, 20,000, 10,000, 6000, 5000, 4000, 2000, 1000, 800, 500, 200, 100, 60, 50, 30, 20, or 10 nm, and at most 10% can have a diameter less than 20,000, 10,000, 6000, 5000, 4000, 2000, 1000, 800, 500, 200, 100, 60, 50, 30, 20, 10, or 5 nm.

The FNP process uses micromixing geometries to mix an incoming, miscible solvent stream in which a polymer is dissolved (so that it can also be termed a polymer solution stream) with a non-solvent stream to produce supersaturation levels as high as 10,000 with mixing times of about 1.5 ms. For example, supersaturation levels can range from about 100, 300, 1000, 3000, 10,000, 30,000, 100,000, or 300,000 to about 300, 1000, 3000, 10,000, 30,000, 100,000, 300,000, or 1,000,000, and mixing times can range from about 0.01, 0.03, 0.1, 0.3, 1, 1.5, 3, 10, 15, 30, 100, or 300 ms to about 0.03, 0.1, 0.3, 1, 1.5, 3, 10, 15, 30, 100, 300, or 1000 ms. It is desirable that these mixing times are shorter than the nucleation and growth times of nanoparticle assembly, so that the size of the nanoparticles formed is constrained. The solvent stream and non-solvent stream can be further mixed with a collection solution, for example, a collection solution that includes a stabilizer such as an amphiphilic surfactant molecule. Nanoparticles can be formed for a variety of pharmaceutical compound, imaging agent, security ink, and drug targeting applications (Johnson, B.K. et al., Chemical processing and micromixing in confined impinging jets, AIChE J. Sep 2003, 49(9), 2264-2282; Johnson B.K. et al., Mechanism for rapid self-assembly of block copolymer nanoparticles, Phys. Rev. Lett. Sep 12, 2003, 91(11); Johnson, B.K. et al.. Flash NanoPrecipitation of organic actives and block copolymers using a confined impinging jets mixer, Australian J. Chem. 2003, 56(10), 1021-1024; Johnson, B.K. et al., Nanoprecipitation of organic actives using mixing and block copolymer stabilization, Abstracts of Papers of the American Chemical Society Sep 2003, 226, U487-U487; Johnson B.K. et al., Engineering the direct precipitation of stabilized organic and block copolymer nanoparticles as unique composites, Abstracts of Papers of the American Chemical Society Sep 2003, 226, U527-U527; Johnson, B.K. et al., Nanoprecipitation of pharmaceuticals using mixing and block copolymer stabilization, Polymeric Drug Delivery II: Polymeric Matrices and Drug Particle Engineering 2006, 924, 278-291; Ansell, S.M. et al., Modulating the therapeutic activity of nanoparticle delivered paclitaxel by manipulating the hydrophobicity of prodrug conjugates, J. Med. Chem. Jun 2008, 51(11), 3288-3296; Gindy, M.E. et al.. Preparation of Poly(ethylene glycol) Protected Nanoparticles with Variable Bioconjugate Ligand Density, Biomacromolecules Oct 2008, 9(10), 2705-2711; Gindy, M.E. et al., Composite block copolymer stabilized nanoparticles: Simultaneous encapsulation of organic actives and inorganic nanostructures, Langmuir Jan 2008, 24(1), 83-90; Akbulut M. et al., Generic Method of Preparing Multifunctional Fluorescent Nanoparticles Using Flash NanoPrecipitation, Adv. Funct. Mater. 2009, 19, 1-8; Budijono, S.J. et al., Exploration of Nanoparticle Block Copolymer Surface Coverage on Nanoparticles, Colloids and Surfaces A -Physicochemical and Engineering Aspects, 2010; Budijono, S.J. et al., Synthesis of Stable Block-Copolymer-Protected NaYF4:Yb3+, Er3+ Up-Converting Phosphor Nanoparticles, Chem. Mat. 2010, 22(2), 311-318; D'Addio, S.M. et al., Novel Method for Concentrating and Drying Polymeric Nanoparticles: Hydrogen Bonding Coacervate Precipitation, Molecular Pharmaceutics Mar-Apr 2010, 7(2), 557-564; Kumar, V. et al., Fluorescent Polymeric Nanoparticles: Aggregation and Phase Behavior of Pyrene and Amphotericin B Molecules in Nanoparticle Cores, Small Dec 2010, 6(24), 2907-2914; Kumar, V. et al., Stabilization of the Nitric Oxide (NO) Prodrugs and Anticancer Leads, PABA/NO and Double JS-K, through Incorporation into PEG-Protected Nanoparticles, Molecular Pharmaceutics Jan-Feb 2010, 7(1), 291-298; D'Addio, S.M. et al., Controlling drug nanoparticle formation by rapid precipitation, Adv. Drug Delivery Rev. May 2011, 63(6), 417-426; Kumar, V. et al., Fluorescent Polymeric Nanoparticles: Aggregation and Phase Behavior of Pyrene and Amphotericin B Molecules in Nanoparticle Cores, Small Dec 2011, 6(24), 2907-2914; Shan, J.N. et al., Pegylated Composite Nanoparticles Containing Upconverting Phosphors and meso-Tetraphenyl porphine (TPP) for Photodynamic Therapy, Adv. Functional Materials Jul 2011, 21(13), 2488-2495; Shen, H. et al., Self-assembling process of flash nanoprecipitation in a multi-inlet vortex mixer to produce drug-loaded polymeric nanoparticles, J. Nanoparticle Res. Sep 2011, 13(9), 4109-4120; Zhang, S.Y. et al., Photocrosslinking the polystyrene core of block-copolymer nanoparticles, Polym. Chem. Mar 2011, 2(3), 665-671; Zhang, S.Y. et al., Block Copolymer Nanoparticles as Nanobeads for the Polymerase Chain Reaction, Nano Lett. Apr 2011, 11(4), 1723-1726; D'Addio, S.M. et al., Constant size, variable density aerosol particles by ultrasonic spray freeze drying, Int'l J. Pharmaceutics May 2012, 427(2), 185-191; D'Addio, S.M. et al., Effects of block copolymer properties on nanocarrier protection from in vivo clearance, J. Controlled Release Aug 2012, 162(1), 208-217; D'Addio, S.M. et al., Optimization of cell receptor-specific targeting through multivalent surface decoration of polymeric nanocarriers, J. Controlled Release May 2013, 168(1), 41-49; Figueroa, C.E. et al., Effervescent redispersion of lyophilized polymeric nanoparticles, Therapeutic Delivery 2013, 4(2), 177-190; Figueroa, C.E. et al., Highly loaded nanoparticulate formulation of progesterone for emergency traumatic brain injury treatment, Therapeutic Delivery 2013, 3(11), 1269-1279; Pinkerton, N.M. et al., Formation of Stable Nanocarriers by in Situ Ion Pairing during Block-Copolymer-Directed Rapid Precipitation, Mol. Pharmaceutics 2013, 10, 319-328; Pinkerton, N.M. et al., Gelation Chemistries for the Encapsulation of Nanoparticles in Composite Gel Microparticles for Lung Imaging and Drug Delivery, Biomacromolecules 2013; DOI: 10.1021/bm4015232). Flash NanoPrecipitation can be used with stabilizing block copolymers to produce nanoparticles. Alternatively, FNP can be used for the production of polystyrene particles without an added stabilizer or amphiphilic copolymer. Nanoparticles over the size range of 60 to 200 nm with polydispersities comparable to those produced by emulsion polymerization were obtained using only electrostatic stabilization.

FNP overcomes the limitations of previous approaches that did not control the size of the assembled nanoparticles, were unable to produce nanoparticles with controlled stoichiometry, and were slow and not scalable. With FNP, nanoparticle size can be controlled. Rapid micromixing to a uniform high supersaturation produces diffusion limited aggregation, and the aggregating solutes or polymers "stick" randomly to each other, so that each particle contains the stoichiometric ratio of solutes that are introduced into the FNP micromixer. Although the process is random, because each nanoparticle contains polymer chains on the order of 50,000 Da molecular weight, the variance in concentration between particles is small. The FNP process takes on the order of 15 ms for particle formation. The FNP process has been scaled to 1400 kg/day by BASF.

Thus, FNP is a room temperature, low energy, one-step, rapid, and continuous route to produce polymer-polymer Janus nanoparticles. A schematic of the FNP process is illustrated in **Figure 1****.** The mixing occurs in a central cavity **3** fed by two incoming streams **1** and **2** that are high velocity linear jets of fluid. The one stream **1** contains the polymers dissolved in a solvent. The other stream **2** is of a non-solvent for the polymer. The compositions and ratios of the streams are chosen so that after mixing in the central cavity **3,** the polymers are no longer dissolved and rapid precipitation occurs (Johnson, B.K. et al., AIChE J. 2003, 49, 2264; Johnson, B.K. et al, Phys. Rev. Lett. 2003, 91; Johnson, B.K. et al., Aust. J. Chem. 2003, 56, 1021; Pustulka, K.M. et al., Mol. Pharmaceutics, 2013, 10, 4367). The nanoparticles formed **4** can be collected in a collection solution **5.** Different mixing geometries can be used in this process, as long as the selected mixing geometry selected produces rapid micromixing to control precipitation (Burke, P.A. et al., international patent application PCT/US2011/031540 and U.S. published patent application US20130037977). The polymer solution rapidly mixes with the non-solvent for a few milliseconds to induce self-assembly of the polymers into kinetically frozen nanoparticles. When used to form polymeric Janus particles two polymers may be dissolved in the solvent (e.g., an organic solvent) of stream 1. However, other hydrophobic components such as small molecule drugs, imaging agents, particles, and therapeutic agents can be successfully encapsulated into polymeric nanoparticles by FNP (Shan, J. et al, Adv. Funct. Mater. 2011, 21, 2488.; Kumar, V. et al., Small 2010, 6, 2907; Pinkerton, N.M. et al., Biomacromolecules 2014, 15, 252). A wide range of solvents and non-solvents that are miscible can be used in the process. Solvents include materials in which the polymer components are soluble. The solvent is miscible with the non-solvent. Nonsolvents include materials in which the polymer components are not soluble or are only sparingly soluble. For example, the solvent can be a non-aqueous solvent, such as an organic solvent or a low polarity solvent, and the non-solvent can be water, a predominantly aqueous phase, or a high polarity solvent. Alternatively, the solvent can be water or a high polarity solvent (for example, if the polymer to be dissolved is a hydrophilic polymer) and the non-solvent can be a non-aqueous solvent or a low polarity solvent. Alternatively, the solvent and the non-solvent can be selected from two different non-aqueous solvents. The solvent or the non-solvent can be polar or nonpolar (or have an intermediate polarity) and can be protic or aprotic. Examples of materials that can be used as solvents or non-solvents include water, alcohols, such as methanol, ethanol, isopropanol (2-propanol), and n-propanol (1-propanol), carboxylic acids, such as formic acid, acetic acid, propanoic acid (propionic acid), butyric acid, furans, such as tetrahydrofuran (THF), dioxane, 1,4-dioxane, furfuryl alcohol, ketones, such as acetone and methyl ethyl ketone (MEK), other water-miscible solvents, such as acetaldehyde, ethylene glycol, propanediol, propylene glycol (propane-1,2-diol), 1,3-propanediol, butanediol, 1,2-butanediol, 1,3-butanediol, 1,4-butanediol, pentanediol, 1,5-pentanediol, 2-butoxyethanol, glycerol, triethylene glycol, dimethyl sulfoxide (DMSO), ethylamine, diethanolamine, diethylenetriamine, methyl diethanolamine, dimethylformamide (DMF), and pyridine, acetonitrile, methyl isocyanide, esters, such as methyl acetate and ethyl acetate, ethers, such as diethyl ether and dimethoxyethane, carbon disulfide, halogenated organics, such as carbon tetrachloride, alkanes, such as heptane, alkenes, such as hexene, cycloalkanes, such as cyclohexane, aromatic hydrocarbons, such as toluene, other organic and inorganic materials, and mixtures of these. A further description of solvent compositions useful for processing by FNP has been presented in B.K. Johnson, R.K. Prud'homme, US Patent Application Pub. US 2012/0171254 A1, July 5, 2012.

FNP is useful in producing homogenous nanoparticles of various polymers including polystyrene, polymethylmethacrylate, and polycaprolactone with controlled diameters and narrow polydispersity indexes (PDIs) (Kumar V. et al., Preparation of lipid nanoparticles: Google Patents, 2013 (EP2558074)). Neither premade nanoparticles nor immobilization steps are required for the FNP process. By simply adjusting the initial polymer concentrations, it is possible to tune the anisotropy of the Janus nanoparticles. Hybrid polymer-inorganic Janus nanoparticles can be made by the FNP process. FNP has been previously described in the following patent documents:
*Preparation of Lipid Nanoparticles,* M. Gindy, et al., US Patent Publication, US20130037977 A1, PCT/US2011/031540, publication date Feb 14, 2013;
*A high-loading nanoparticle-basedformulationfor water-insoluble steroids,* C. Figureroa et al., Patent Publication, WO2013063279 A1, PCT/US2012/061945, publication date May 2, 2013;
*Particulate constructs for release of active agents,* L.D. Mayer et al., Patent Publication US20130336915 A1, Publication date Dec. 19, 2013; and
Process and Apparatuses for Preparing Nanoparticle Compositions with Amphiphilic Copolymers and Their Use, B.K. Johnson et al., US Patent Application Pub., US 2012/0171254 A1, July 5, 2012.
The production of single component polymer nanoparticles by FNP has been described in Zhang, C. et al., Flash nanoprecipitation of polystyrene nanoparticles, Soft Matter 2012, 8(1), 86-93:

The FNP process requires adequate micromixing, which has been described in the patents above. FNP requires that the polymers or inorganic colloids of interest be mutually soluble in a common organic process solvent which is miscible with the non-solvent stream. Water or an aqueous solution can be used as the non-solvent stream and a water-miscible organic solvent can be used as the process solvent stream. With the polymer additives the convergence of the two streams produces a dispersed Janus nanoparticle dispersion in the mixed solvent phase.

The FNP process may be run without a stabilizer additive, so that the process solvent contains the polymers and/or colloids of interest without an amphiphilic stabilizer. Alternatively, amphiphilic stabilizers may be added to either the process solvent phase or the non-solvent phase. It is also possible to reverse the solvent polarity and to precipitate water soluble Janus particles in a non-aqueous non-solvent phase.

Particles may be produced by the FNP process to have, for example, diameters between 10 nm and 4000 nm, between 20 nm and 1000 nm, or between 50 nm and 800 nm. The sizes are the intensity weighted average size determined by dynamic light scattering. Such measurements can be conducted in a Malvern Nanosizer dynamic light scattering (DLS) instrument. The size reported by dynamic light scattering is the intensity weighted diameter, which is used herein to report sizes of the particles produced by the Flash NanoPrecipitation process. The breadth of distribution of the particle diameters can be characterized by values such as the Di90, the intensity-weighted diameter where 90% of the particles have a lesser diameter, the Di50, the intensity-weighted diameter where 50% of the particles have a lesser diameter, and the Di10, the intensity-weighted diameter where 10% of the particles have a lesser diameter. For example, to define a minimum narrowness of distribution of particle diameters, it can be specified that at least 90% of the particles have a diameter less than a nominal Di90 value and that at most 10% of the nanoparticles formed have a diameter less than a nominal Di10 value, or that 80% of the nanoparticles have a diameter greater than or equal to the nominal Di10 value and less than the nominal Di90 value. Alternatively, the Span can be defined as the difference between the Di90 and Di10 values divided by the Di50 value, that is, Span = (Di90-Di10)/Di50. A smaller Span indicates a more narrow distribution of particle sizes, with a Span of zero indicating a monodisperse distribution (i.e., all particles have the same size. The Di10, Di50, and Di90 values are determined from the intensity weighted distribution that is obtained from the dynamic light scattering measurement. These values can be calculated on a mass-weighted basis using standard conversions from intensity-to mass-weighted distributions.

### Stabilizers and their removal

Stabilizers that have been incorporated into the FNP process may need to be substantially removed from the final Janus formulation, depending on the application of the particles.

In a first approach, if the stabilizers have a solubility in the external phase of greater than, for example, 10⁻³ wt%, then they can be removed by solvent exchange and diffusion. This can be done by batch dialysis, by tangential flow ultrafiltration, by centrifugation and decanting, or other processes for removing soluble impurities from particulate suspensions. Once the amphiphilic stabilizer is removed the intrinsic properties of the polymers comprising the Janus core will be exposed.

A second approach involves specific complexation of the surfactant to remove it from the particle surface. One such example is the complexation of surfactants, notably sodium dodecyl sulfate (SDS), using cyclodextrins. The binding constant of SDS to cyclodextrin has a higher affinity than binding to the particle surface. Thus, the surfactant can be removed from the surface. This process has been described for the removal of SDS from hydrophopic associative polymers, and has been used in the synthesis and purification of copolymers using cyclodextrins. The SDS:cyclodextrin complex is stable in the particle dispersion, but it may be desirable to remove the soluble SDS:cyclodextrin complex by one of the methods presented above. SDS is a representative interfacial stabilizer for either FNP or emulsion processing, but other surfactants strongly binding with cyclodextrin can also be used.

In some FNP processes (according to invention) and emulsion processes (not according to invention) it may be desirable to use larger amphiphilic polymers, block copolymers, or surfactants whose solubility is so low that they cannot be removed by the processes described above. For this, a third approach is to use cleavable amphiphilic stabilizers in which the hydrophilic moiety in the stabilizer is attached to the hydrophobic moiety by a linker that may be broken or cleaved. The result is that the hydrophobic moiety is left on the surface and the hydrophilic moiety is dispersed in the external aqueous phase. For an external hydrophobic solvent phase the system is reversed. Cleavable linkages may be esters, orthoesters, ketal, disulfides, or other groups well known in the field that are cleaved by hydrolysis, redox reactions, exchange reactions, enzymatic attack, or other chemical or biochemical reactions that can be initiated by changes in pH, redox conditions, or the addition of catalytic species. In some cases, this third approach using cleavable amphiphilic stabilizers may be desirable to produce nanoparticles that do not contain amphiphilic components in the final formulation, the cleaving process renders the stabilizer no longer amphiphilic.

If the amphiphilic anchoring species is dilute enough on the particle surface, then its presence on the surface will not alter the desirable Janus surface properties.

In another embodiment, when the amphiphilic anchoring block is high enough in molecular weight, for example, above 900 Daltons, it can become part of the Janus phase. For example, an FNP or emulsion process can be conducted with an amphiphilic block copolymer having the hydrophobic block of the same type as the material composing the Janus particle. After Janus particle formation the amphiphilic anchoring block is cleaved removing the soluble portion from the particle surface, while leaving the anchoring block anchored in the polymer matrix. Two or more amphiphilic stabilizers can be used, each with the hydrophobic block being that of one of the polymer phases in the Janus core. The amphiphilic stabilizers will partition on the particle surface, driven by the enthalpic energy of phase separation, which drives the phase separation of the Janus core. Once the soluble components are cleaved the Janus particle will have separate phases which now include the component from the amphiphilic stabilizer. The ratio of amphiphilic stabilizers is adjusted to approximately reflect the volume ratio of the Janus particle core. For example, if the Janus particle is to have a 50:50 ratio of two polymers, the stabilizers should be used in approximately a 50:50 ratio. The exact ratio depends on both the size of the hydrophobic and the hydrophilic blocks to create an optimal surface area ratio.

### Isolation of Janus particles

Post processing steps can be applied to the particle phases to concentrate the Janus particles, remove residual process solvent, change the process solvent, or change the non-solvent. Concentration can be effected by ultrafiltration, selective flocculation, for example, as described by D'Addio, S.M. et al., Novel Method for Concentrating and Drying Polymeric Nanoparticles: Hydrogen Bonding Coacervate Precipitation. Molecular Pharmaceutics Mar-Apr 2010, 7(2), 557-564, centrifugation, freeze drying, spray drying, or tray or drum drying. Excipients may be added during the drying or concentrating phases to minimize Janus particle aggregation, or to enhance redispersion. For example, polyethylene glycol from 1000 to 20,000 molecular weight can be used as an excipient.

### Organic-inorganic Janus particles

A Janus structure may contain two or more polymer phases and an inorganic material may be imbedded in one of the polymer phases, or it may be deposited or reacted on the surface of one of the Janus faces, or it may be reacted into the bulk of one of the Janus polymer phases. The reaction steps can occur during or after the particle formation step.

The physical self-assembly of hybrid inorganic/polymer nanocomposites previously required pre-synthesized nanoparticles with well-defined surface chemistry. However, the flash nanoprecipitation process of the current invention allows for the self-assembly of hybrid organic-inorganic nanoparticles in one step. FNP can fabricate nanoparticles composed of inorganic metals and organic polymers, and the process may be further extended to the fabrication of hybrid particles containing other inorganic nanomaterials such as crystals in addition to polymers. Spherical polyelectrolyte brushes (SPBs) can be used for the generation and stabilization of metallic nanoparticles. These SPBs can consist of a solid polystyrene (PS) core onto which long cationic polyelectrolyte chains are grafted. The dense layer of polyelectrolyte chains confined on the surface of the core particles can be used to immobilize metal ions. Reduction of these immobilized metal ions with a reducing agent, such as sodium borohydride (NaBH₄), can lead to nanoparticles of the respective metal. Other reducing agents that can be used include lithium aluminum hydride (LiAlH₄), compounds containing the Sn²⁺ ion, such as tin(II)chloride (SnCl₂), compounds containing the Fe²⁺ ion, such as iron(II)sulfate (FeSO₄), oxalic acid, formic acid, ascorbic acid, sulfite compounds, phosphites, hydrophosphites, and phosphorous acid, dithiothreitol (DTT), and tris(2-carboxyethyl)phosphine HCl (TCEP), carbon, carbon monoxide (CO), diisobutylaluminum hydride (DIBAL-H), Lindlar catalyst, sodium amalgam, zinc amalgam (Clemmensen reduction), diborane, hydrazine (Wolff-Kishner reduction), and nascent (atomic) hydrogen.

The whole process can involve multiple steps of synthesis of core-shell polymeric nanoparticles, adsorption of metal ions, and reduction and stabilization of metal nanoparticles.

A one-step synergistic preparation of metal nanoparticles within spherical polymer nanoparticles through Flash NanoPrecipitation (FNP) was carried out. FNP can generate monodisperse polystyrene nanoparticles and illustrated that the sizes of PS nanoparticles can be fine-tuned between 30 and 150 nm by changing the polymer and/or electrolyte concentration (Zhang, C. et al., Soft Matter 2012, 8, 86; Chung, J.W., J. Colloid Interface Sci. 2013, 396, 16). Advantages of FNP are that it is a three-component process (involving only bulk polymer, a solvent, and a non-solvent) with low residence time, continuous processing, low energy consumption, and high reproducibility.

Polystyrene-block-poly(vinylpyridine) (PS-b-PVP) in THF, aqueous metal ion salts, and reducing agent solutions are employed as polymer stream, non-solvent stream and collection solution, respectively. Uniform metal nanoparticles grown on polymer nanospheres are obtained through a one-step FNP process. The particle size and metal nanoparticle loading amount can be tuned by changing the preparation parameters (e.g., feeding amount, feeding speed, and mixing rate).

FNP can fabricate nanoparticles composed of inorganic metals and organic polymers. Not forming part of this invention, the process may be further extended to the fabrication of hybrid particles containing other inorganic nanomaterials such as crystals in addition to polymers. The process is applicable to many metals and mixtures of metals. The inorganic may be deposited as a metal, or as a metal or mixed metal oxide. Many reducing agents may be employed in the reduction step, and may be introduced during the FNP process or, in some cases, added later to the reaction bath. In comparison to existing processes, the one-step FNP technique is a single-step, low energy, continuous, and rapid process.

The FNP route to metal-polymer nanoparticles is a one-step process with a short processing time. This approach allows for the development of a range of hybrid nanoparticles with varying structural features that are difficult to achieve by conventional multi-step synthetic approaches. Examples of metals include gold (Au), platinum (Pt), silver (Ag), palladium (Pd), copper (Cu), cobalt (Co), and iron (Fe). Other metals and combinations of these can be used. For example, metals and combinations of metals that have catalytic properties can be used. Block copolymers in which one block is composed of a polyelectrolyte (e.g., PS-co-PVP) are suitable.

Metal-polymer nanoparticles are useful in nanotechnology applications, including single nanoreactors, catalyst supports, adsorbents, drug delivery vehicles, medical imaging agents, emulsion stabilization and chemical reactivity agents for emulsion-based reaction processes, large-scale composite nanomaterials, sensors and electronic devices (Xu, P. et al., Chem. Soc. Rev. 2014, 1349). Thus, metal-polymer nanoparticles can find application in the waste water treatment, battery, oil industry, and medical sectors.

Two challenges with this technology are controlling the stability of the particles in aqueous solution and the distribution of particle sizes that broaden as the concentration of polymers in the input stream is increased. Both of these challenges, though, may be overcome by optimizing the particle processing conditions or by incorporating stabilizing agents such as surfactants in the nanoparticle fabrication process.

The process according to the invention of producing metal-polymer nanoparticles is facile and rapid compared to other approaches. Currently, metal-polymer nanoparticles are not commercially available. Previously, metal-polymer nanoparticles could not be made in a scalable, cost-efficient way.

### Example 5: Metal-polymer nanoparticles

A process according to the invention generates metal-polymer nanoparticles through a one-step, self-assembly approach. That is, the self-assembly process is simplified by synergistically preparing hybrid metal nanoparticle deposited onto spherical polymer assemblies, namely, metal on polystyrene-*b*-poly(4-vinylpyridine) (metal-PS-*b*-PVP) through the one-step continuous route of Flash NanoPrecipitation (FNP). Polystyrene-block-poly(vinylpyridine) (PS-b-PVP) in THF, aqueous gold salts, and reducing agent solutions are employed as the polymer stream, non-solvent stream, and collection solution, respectively. Uniform Au nanoparticles deposited on polymer nanospheres are then obtained through a one-step FNP process. Stabilizers, such as sodium dodecyl sulfate (SDS), may be added to the process, for example, to the collection solution, to further control stability.

A confined impinging jet mixer composed of two separate streams was used. A syringe containing 1 mL of 3 mg/mL PS₇₉₃ₖ-b-PVP₃ₛₖ (PDI= 1.08, purchased from Polymer Source, Inc.) in THF was placed at the inlet of Stream 1, and a syringe containing 1 mL of 0.45 mg/mL chloroauric acid (HAuCl₄) in H₂O was placed at the inlet of Stream 2. Subsequently, fluid was expelled manually from both syringes at the same rate (~1 mL per second), causing the two streams to merge into a mixing stream. The mixing stream was diluted into a 10 mL water reservoir containing 1 mg of the reducing agent, sodium borohydride (NaBH₄), and 10 mg of the stabilizer, sodium dodecyl sulfate (SDS). The water reservoir quenched the precipitated nanoparticles, and a stable colloid solution was formed. In a separate experiment, Pt@PS-b-PVP was prepared by replacing Stream 2 with 1 mL of 3 mg/mL hexachloroplatinic acid (H₂PtCl₆) in water (H₂O).

Scanning electron microscopy (SEM) shows that the obtained Au@PS-*b*-PVP (P1) composites have regular spherical morphology with a uniform diameter of ~80 nm. The dynamic light scattering (DLS) data and a photograph of colloid solution show that the obtained composites are well dispersed in water with hydrodynamic diameter ~100 nm. A transmission electron microscopic (TEM) image confirmed that the Au@PS-*b*-PVP nanoparticles are monodisperse and uniformly spherical, with a mean diameter of ∼80 nm. Furthermore, the characteristics of the individual nanoparticles were visualized by TEM. The Au@PS-*b*-PVP nanoparticle consists of a predominantly PS core, a thin PVP shell and Au nanocrystals (NCs) uniformly embedded in the PVP layer. The diameter of Au NCs was about 3 nm and the 0.23 nm lattice spacing corresponding to the (111) plane of face-centered cubic (fcc) structure of gold nanoparticles was visually confirmed. All the Au NCs were located near the surface of the polymeric support, and aggregation of the Au NCs on the surface was minimal.

In order to confirm that the PVP segment of the PS-b-PVP block copolymer contributes to the formation of the composite, a control experiment was carried out by using PS homopolymer (Mw = 376 kg/mol) dissolved in THF as Stream 1. A large amount of free Au NCs are formed while no Au NCs are observed on the surface of the polymer nanoparticles. The above observation and comparison indicate that the AuCl₄^{―} ions in the system strongly localize to and disperse well within the PVP corona of the polymer particles due to the complexation of the AuCl₄^{―} ions with the pyridine units of the PVP blocks (Spatz, J. P. et al., Langmuir 2000, 16, 407; Spatz, J.P. et al., Adv. Mater. 2002, 14, 1827; Suntivich, P. et al., Langmuir 2011, 27, 10730; Leong, W.L. et al., Adv. Mater. 2008, 20, 2325).

Without being bound by theory, the process of nanoparticle formation can be envisioned as follows. PS-*b*-PVP block polymers self-assemble into nanoparticles with PS as the predominant core and PVP as the corona when the two input streams mix in the confined chamber. Subsequently, AuCl₄^{―} ions are attracted into the PVP network. The ion-block copolymer complex disperses into a water reservoir containing NaBH₄ that reduces the entrapped AuCl₄^{―} ions into Au seeds. The growth of gold within the PVP layer then takes place and the complex is quenched to form stable Au@PS-*b*-PVP composites. The presence of SDS in the water reservoir inhibits particle coalescence while the absence of SDS results in nanoparticle aggregation within minutes. The reported methodology has also been demonstrated in the successful fabrication of Pt@PS-*b*-PVP nanoparticles when using H₂PtCl₆ in H₂O as Stream 2: ~2 nm Pt nanocrystals uniformly deposit on the surface of the polymer nanoparticles.

The effect of processing parameters on the preparation of the composites was studied by varying polymer and gold salt concentrations, as shown in Table 2 below.

**Table 2**

| | **Stream 1 PS-b-PVP /THF (mg/mL)** | **Stream 2 HAuCl₄/H₂O (mg/mL)** |
|---|---|---|
| **P1** | 3 | 0.45 |
| **P2** | 3 | 0.15 |
| **P3** | 3 | 0.9 |
| **P4** | 6 | 0.45 |
| **P5** | 1 | 0.45 |
| **P6** | 1 | 0.15 |

The composites P2 and P3 were prepared by feeding 0.15 mg/mL and 0.9 mg/mL HAuCl₄ concentrations, respectively, in Stream 2. The two composites along with P1, created from 0.45 mg/mL HAuCl₄ feed concentration, have nearly the same composite diameter and size distribution independent of the Au salt concentration in the feed. This was also confirmed by DLS measurements. Increasing the HAuCl₄ feed concentration to 0.9 mg/mL produced a denser distribution of Au nanocrystals (NCs) on the polymer surface and slightly increased the Au NC sizes to ∼5 nm. Lowering the HAuCl₄ feed concentration to 0.15 mg/mL resulted in a sparser distribution of Au nanocrystals. The UV-Vis absorbance of the composites was measured and investigated for the surface plasmon resonance (SPR) peak associated with the presence of Au nanoparticles. The spectrum of P2 did not show an obvious plasmon band, indicating a discrete distribution of Au NCs. As the HAuCl₄ concentration in the feed was increased, a sharper and more intense plasmon absorption band with a slight red shift is observed, which was thought to result from the larger Au nanocrystals as well as the reduced Au nanoparticle interspacing. (Jana, N.R. et al., Langmuir 2001, 17, 6782.; Hussain, 1. et al., J. Am. Chem. Soc. 2005, 127, 16398; Rao, T.L. et al., Soft Matter 2012, 8, 2963.)

The overall size of the composites, on the other hand, was controlled by varying the PS-*b*-PVP concentration in the THF stream. **Figures 5A and 5B** show a TEM image and DLS data, respectively, of P4 particles prepared with 6 mg/mL polymer concentration. As the polymer concentration increased, the nanoparticles also increased in size to ~120 nm while remaining monodisperse. Similarly, lowering the feed polymer concentration to 1 mg/mL reduced the size of P5 to ~30 nm. The large Au clusters in P5 are formed on the polymer surface due to the higher local concentration of gold salts per polymer nanoparticle. Decreasing the feed polymer and gold salt concentrations to 1 mg/mL and 0.15 mg/mL, respectively, led to ~30 nm nanoparticles (P6) with a distribution of well-dispersed Au NCs on the surface, as shown by the TEM image and DLS data of **Figures 5C and 5D****,** respectively. Some polymer composite aggregates form in P5 and P6 at a polymer concentration of 0.15 mg/mL regardless of gold salt concentration, which led to a large hydrodynamic diameter of ~80 nm.

### Example 6: Catalysis with metal-polymer nanoparticles

The gold-catalyzed reduction of 4-nitrophenol by NaBH₄ to 4-aminophenol was used as a model reaction to evaluate the catalytic capability of the synthesized Au@PS-*b*-PVP hybrid nanoparticles (P1). 1 mL of 0.1 mM 4-nitrophenol was mixed with a freshly prepared aqueous solution of NaBH₄ (2 mL, 0.1 M). Au@PS-b-PVP (P1) (250µg) was then added. UV/Vis absorption spectra were recorded to monitor the change in the reaction mixture after the removal of nanoparticles.

The reduction reaction did not proceed without the presence of Au@PS-*b*-PVP catalyst, as evidenced by a constant absorption peak at 400 nm. However, when Au@PS-*b-*PVP catalyst was introduced into the solution, the absorption at 400 nm quickly decreased while the absorption at 295 nm increased. The reduction of 4-nitrophenol into 4-aminophenol was completed in ~1 min. The complete conversion of 4-nitrophenol could also be visually appreciated by the color change of the solution from yellow to clear.

Stability against coalescence is an important issue for nanocrystal-based catalysts. (Comotti, M. et al., Angew. Chem. 2004, 116, 5936; Angew. Chem. Int. Ed. 2006, 45, 8224; Valdés-Solis, T. et al., J. Catal. 2007, 251, 239.) The stability of Au@PS-*b*-PVP was investigated by repeating the reduction reaction with the same catalyst for five cycles. After each reaction, the catalyst was recycled by centrifugation, followed by washing with distilled water and drying in vacuum overnight at room temperature. The catalyst showed high activity after five successive cycles of reactions, with conversion close to 100% within ~1 min of reaction time. The well-dispersed Au NCs were still visualized by TEM on the surface of the polymer support after five reaction cycles. Thus, the composites with gold nanoparticles embedded in the corona were effective at preventing the coalescence of catalyst nanoparticles, making the catalyst reusable after multiple cycles of reactions. Combining the advantages of facile synthesis and large scale production, FNP is an attractive platform for the production of stable and recyclable nanocatalysts.

In summary, hybrid Au (or Pt) on PS-b-PVP assemblies were produced through Flash NanoPrecipitation. Control over the nanoparticle size, gold cluster size, and overall optical response was achieved by changing the process parameters. The hybrid nanoparticles exhibited high catalytic performance and good reusability for the reduction of 4-nitrophenol.

### References

Sharma G. et al., Macromol. Rapid Commun. 2004, 25, 547.
Mei, Y. et al., Langmuir 2005, 21, 12229.
Budijono, S. et al., Nanoparticles for photodynamic therapy: Google Patents; 2009 (PCT/US2008/012485 and US20110022129).
Burke, P.A. et al., Preparation of lipid nanoparticles: Google Patents; 2011 (PCT/US2011/031540 and US20130037977).
Kumar, V. et al., Preparation of lipid nanoparticles: Google Patents; 2013 (EP2558074).
Mayer, L.D. et al., Particulate constructs for release of active agents: Google Patents; 2006 (PCT/US2005/025549).
Mayer, L.D. et al., Particulate constructs for release of active agents: Google Patents; 2007 (EP1786443).
Prud'homme, R.K. et al., Lung targeting dual drug delivery system: Google Patents; 2011 (US20110268803).
York, A.W. et al., Bioactive amphiphilic polymer stabilized nanoparticles with enhanced stability and activity: Google Patents; 2013 (PCT/US2012/050040).
Gindy, M. et al., Preparation of Lipid Nanoparticles, US Patent Publication, US20130037977 A1, PCT/US2011/031540, publication date Feb 14, 2013.
Figureroa, C. et al., A high-loading nanoparticle-based formulation for water-insoluble steroids, Patent Publication, WO2013063279 A1, PCT/US2012/061945, publication date May 2, 2013.
Mayer, L.D. et al., Particulate constructs for release of active agents, Patent Publication US20130336915 A1, publication date Dec. 19, 2013.
Johnson, B.K. et al., Process and Apparatuses for Preparing Nanoparticle Compositions with Amphiphilic Copolymers and Their Use, US Patent Application Pub., US 2012/0171254 A1, July 5, 2012.

## Claims

1. A method for forming a metal-polymer composite nanoparticle, comprising
dissolving a polymer in a first solvent at a first concentration to form a polymer solution (1),
dissolving a metal salt in a second solvent at a second concentration to form a metal salt solution (2), and
through flash nanoprecipitation (3) mixing the polymer solution with the metal salt solution to form
the metal-polymer composite nanoparticle having a surface (4),
wherein the metal is concentrated at the surface, and
wherein the second solvent is a nonsolvent for the polymer,
wherein the polymer is an amphiphilic block copolymer, and
wherein the polymer is polystyrene-block-poly(vinylpyridine), wherein the metal is selected from the group consisting of gold, platinum, silver, palladium, copper, cobalt, iron, and combinations, wherein the mixing of the polymer solution with the metal salt solution further comprises mixing with a collection solution (5), wherein the collection solution comprises a reducing agent, and wherein the collection solution comprises a stabilizer.

2. The method of claim 1, wherein the reducing agent is selected from the group consisting of lithium aluminum hydride (LiAlH₄), compounds containing the Sn²⁺ ion, compounds containing the Fe²⁺ ion, oxalic acid, formic acid, ascorbic acid, sulfite compounds, phosphites, hydrophosphites, phosphorous acid, dithiothreitol (DTT), tris(2-carboxyethyl)phosphine HCl (TCEP), carbon, and combinations.

3. The method of claim 2, wherein the reducing agent is tin (II) chloride (SnCl₂) or iron (II) sulfate (FeSO₄).

4. The method of claim 1, wherein the reducing agent is sodium borohydride (NaBH₄).

5. The method of claim 1, wherein the stabilizer is sodium dodecyl sulfate (SDS).

6. A metal-polymer composite nanoparticle, comprising
a core and
a shell that surrounds the core,
wherein the core comprises a polymer,
wherein the shell comprises the polymer and a metal,
wherein the polymer is an amphiphilic block copolymer, and
wherein the polymer is polystyrene-block-poly(vinylpyridine), and wherein the metal is selected from the group consisting of gold, platinum, silver, palladium, copper, cobalt, iron, and combinations.

7. A method of using the metal-polymer composite nanoparticle of claim 6 to catalyze a chemical reaction.

8. A method of using the metal-polymer composite nanoparticle of claim 6 to catalyze a chemical reaction between two immiscible phase liquids.

9. The metal-polymer composite nanoparticle of claim 6, wherein the metal is selected from the group consisting of gold (Au), platinum (Pt), silver (Ag), cobalt (Co), and combinations.

## Patentansprüche

1. Verfahren zur Bildung eines Metall-Polymer-Verbund-Nanopartikels, umfassend
das Auflösen eines Polymers in einem ersten Lösungsmittel in einer ersten Konzentration unter Bildung einer Polymerlösung (1);
das Auflösen eines Metallsalzes in einem zweiten Lösungsmittel in einer zweiten Konzentration unter Bildung einer Metallsalzlösung (2); und
Mischen der Polymerlösung mit der Metallsalzlösung durch Flash-NanoPrecipitation (3) unter Bildung
des Metall-Polymer-Verbund-Nanopartikels, das eine Oberfläche (4) aufweist;
wobei das Metall an der Oberfläche konzentriert ist; und
wobei das zweite Lösungsmittel ein Nichtlösungsmittel für das Polymer ist;
wobei das Polymer ein amphiphiles Blockcopolymer ist; und
wobei es sich bei dem Polymer um Polystyrol-Block-Poly(vinylpyridin) handelt, wobei das Metall aus der Gruppe ausgewählt ist, die aus Gold, Platin, Silber, Palladium, Kupfer, Cobalt, Eisen und Kombinationen davon besteht, wobei das Mischen der Polymerlösung mit der Metallsalzlösung weiterhin das Mischen mit einer Sammellösung (5) umfasst, wobei die Sammellösung ein Reduktionsmittel umfasst und wobei die Sammellösung einen Stabilisator umfasst.

2. Verfahren gemäß Anspruch 1, wobei das Reduktionsmittel aus der Gruppe ausgewählt ist, die aus Lithiumaluminiumhydrid (LiAlH₄), Verbindungen, die das Sn²⁺-Ion enthalten, Verbindungen, die das Fe²⁺-Ion enthalten, Oxalsäure, Ameisensäure, Ascorbinsäure, Sulfitverbindungen, Phosphiten, Hydrophosphiten, Phosphoriger Säure, Dithiothreit (DTT), Tris(2-carboxyethyl)phosphin-HCl (TCEP), Kohlenstoff und Kombinationen davon besteht.

3. Verfahren gemäß Anspruch 2, wobei es sich bei dem Reduktionsmittel um Zinn(II)chlorid (SnCl₂) oder Eisen(II)sulfat (FeSO₄) handelt.

4. Verfahren gemäß Anspruch 1, wobei es sich bei dem Reduktionsmittel um Natriumborhydrid (NaBH₄) handelt.

5. Verfahren gemäß Anspruch 1, wobei es sich bei dem Stabilisator um Natriumdodecylsulfat (SDS) handelt.

6. Metall-Polymer-Verbund-Nanopartikel, umfassend
einen Kern und
eine Hülle, die den Kern umgibt;
wobei der Kern ein Polymer umfasst;
wobei die Hülle das Polymer und ein Metall umfasst;
wobei das Polymer ein amphiphiles Blockcopolymer ist; und
wobei es sich bei dem Polymer um Polystyrol-Block-Poly(vinylpyridin) handelt und wobei das Metall aus der Gruppe ausgewählt ist, die aus Gold, Platin, Silber, Palladium, Kupfer, Cobalt, Eisen und Kombinationen davon besteht.

7. Verfahren zur Verwendung des Metall-Polymer-Verbund-Nanopartikels gemäß Anspruch 6, um eine chemische Reaktion zu katalysieren.

8. Verfahren zur Verwendung des Metall-Polymer-Verbund-Nanopartikels gemäß Anspruch 6, um eine chemische Reaktion zwischen Flüssigkeiten, die zwei unmischbare Phasen bilden, zu katalysieren.

9. Metall-Polymer-Verbund-Nanopartikel gemäß Anspruch 6, wobei das Metall aus der Gruppe ausgewählt ist, die aus Gold (Au), Platin (Pt), Silber (Ag), Cobalt (Co) und Kombinationen davon besteht.

## Revendications

1. Procédé de formation d'une nanoparticule composite métal-polymère, comprenant
la dissolution d'un polymère dans un premier solvant à une première concentration pour former une solution de polymère (1),
la dissolution d'un sel de métal dans un second solvant à une seconde concentration pour former une solution de sel de métal (2), et
par le biais d'une nanoprécipitation éclair (3) le mélange de la solution de polymère avec la solution de sel de métal à former
la nanoparticule composite métal-polymère ayant une surface (4),
dans lequel le métal est concentré à la surface, et
dans lequel le second solvant est un non-solvant pour le polymère,
dans lequel le polymère est un copolymère bloc amphiphile, et
dans lequel le polymère est le polystyrène-bloc-poly(vinylpyridine), dans lequel le métal est choisi dans le groupe constitué par l'or, le platine, l'argent, le palladium, le cuivre, le cobalt, le fer, et des combinaisons, dans lequel le mélange de la solution de polymère avec la solution de sel de métal comprend en outre le mélange avec une solution de collecte (5), dans lequel la solution de collecte comprend un agent réducteur, et dans lequel la solution de collecte comprend un stabilisant.

2. Procédé selon la revendication 1, dans lequel l'agent réducteur est choisi dans le groupe constitué par l'hydrure de lithium aluminium (LiAlH₄), des composés contenant l'ion Sn²⁺, des composés contenant l'ion Fe²⁺, l'acide oxalique, l'acide formique, l'acide ascorbique, des composés sulfite, des phosphites, des hydrophosphites, l'acide phosphoreux, le dithiothréitol (DTT), le tris(2-carboxyéthyl)phosphine HCl (TCEP), le carbone, et des combinaisons.

3. Procédé selon la revendication 2, dans lequel l'agent réducteur est le chlorure d'étain (II) (SnCl₂) ou le sulfate de fer (II) (FeSO₄).

4. Procédé selon la revendication 1, dans lequel l'agent réducteur est le borohydrure de sodium (NaBH₄).

5. Procédé selon la revendication 1, dans lequel le stabilisant est le dodécyl sulfate de sodium (SDS).

6. Nanoparticule composite métal-polymère, comprenant
un noyau et
une enveloppe qui entoure le noyau,
dans laquelle le noyau comprend un polymère,
dans laquelle l'enveloppe comprend le polymère et un métal,
dans laquelle le polymère est un copolymère bloc amphiphile, et
dans laquelle le polymère est le polystyrène-bloc-poly(vinylpyridine), et dans laquelle le métal est choisi dans le groupe constitué par l'or, le platine, l'argent, le palladium, le cuivre, le cobalt, le fer, et des combinaisons.

7. Procédé d'utilisation de la nanoparticule composite métal-polymère de la revendication 6 pour catalyser une réaction chimique.

8. Procédé d'utilisation de la nanoparticule composite métal-polymère de la revendication 6 pour catalyser une réaction chimique entre deux liquides de phases immiscibles.

9. Nanoparticule composite métal-polymère selon la revendication 6, dans laquelle le métal est choisi dans le groupe constitué par l'or (Au), le platine (Pt), l'argent (Ag), le cobalt (Co), et des combinaisons.
